(19) Europäisches Patentamt
European Patent Office
Office européen des brevets

(11) Publication number: 0 185 467
A2

(12) **EUROPEAN PATENT APPLICATION**

(21) Application number: 85308378.0

(22) Date of filing: 18.11.85

(51) Int. Cl.[4]: C 08 L 67/02
C 08 L 67/04, A 61 L 17/00

(30) Priority: 19.11.84 US 673073

(43) Date of publication of application:
25.06.86 Bulletin 86/26

(84) Designated Contracting States:
DE FR GB

(71) Applicant: ETHICON INC.
U.S. Route 22
Somerville New Jersey 08876(US)

(72) Inventor: Koelmel, Donald F.
Box 249, Brass Castle Road
Washington New Jersey 07882(US)

(72) Inventor: Shalaby, Shalaby W.
329A Longview Road RD 2
Lebanon, N.J. 08833(US)

(72) Inventor: Jamiolkowski, Dennis D.
20 Fawnridge Drive
Long Valley N.J. 07853(US)

(74) Representative: Jones, Alan John et al,
CARPMAELS & RANSFORD 43 Bloomsbury Square
London, WC1A 2RA(GB)

(54) **Compatible blends of poly(p-dioxanone) and poly(alkylene phenylene-bis-oxyacetate)and absorbable surgical devices made therefrom.**

(57) Single phase blends of polydioxanone and poly(alkylene phenylene-bis-oxyacetate) are disclosed. They are useful in making surgical devices, particularly surgical filaments such as sutures and ligatures.

EP 0 185 467 A2

0185467

# Compatible Blends of Polydioxanone and Poly(alkylene phenylene-bis-oxyacetate) and Absorbable Surgical Devices Made Therefrom

The invention relates to compatible blends of polydioxanone and poly(alkylene phenylene-bis-oxyacetate) and to absorbable surgical devices, especially sutures and ligatures, made therefrom. Despite the dissimilarity in molecular structure between the two polymers (i.e., an all-aliphatic polyester versus a polyester containing a substantial proportion of aromatic groups), the molten blends unexpectedly appear to be single phase compositions, by microscopic examination of the blend in the molten state.

## Background of the Invention

Synthetic absorbable polymers have been used to produce various surgical products such as sutures, implants, prostheses, and the like, for several years. Illustrative U.S. Patents that disclose such polymers are Nos. 3,297,033, 3,044,942, 3,371,069, 3,531,561, 3,636,956, RE 30,170, and 4,052,988.

Implantable surgical devices must be sterile prior to implanting in the body. Sterilization of devices is usually accomplished by the use of heat, ethylene oxide, or gamma radiation using a $^{60}Co$ source. In many cases, the use of gamma radiation is the most convenient and most certain way to effect sterilization. However, all of the synthetic absorbable polymers now in commercial use are degraded at least some extent by gamma radiation. Therefore, unless for some reason degradation of the polymer is desired (for instance, to accelerate the absorption rate),

the use of gamma radiation is ordinarily precluded for the purpose of sterilizing the presently commercial synthetic absorbable polymers.

This invention provides compatible blends of polymers, the blend as a whole being absorbable, that can be sterilized by gamma radiation and still retain a useful level of physical and biological properties. When the blends are fabricated into surgical filaments such as sutures and ligatures, such filaments surprisingly have greater compliance (as evidenced by reduced Young's modulus) than filaments made from either of the two polymeric components alone. When incorporated into molded articles such as surgical staples, the blends impart a desirable level of toughness.

## Summary of the Invention

The single phase (by microscopic examination of the melt) blends of the invention contain polydioxanone and poly(alkylene phenylene-bis-oxyacetate). In a preferred aspect, the blends of the invention are fabricated into drawn and oriented surgical filaments such as sutures and ligatures. In another aspect, the blends of the invention, alone or in combination with other polymeric materials, are employed in surgical staples, e.g., of the fastener/ receiver type.

## Relevant Prior Patent Application

In Koelmel et al., U.S. Patent Application Serial No. 587,332, filed March 7, 1984, for "Poly(p-dioxanone) Polymers Having Improved Radiation Resistance", and assigned to the same assignee as this application, there is disclosed copolyesters produced by reacting p-dioxanone monomer with poly(alkylene phenylene-bis-oxyacetate).

The Prior Art

In Shalaby et al., U.S. Patent No. 4,435,590, there is disclosed radiation sterilizable, absorbable polymers derived from 1,4-phenylene-bis-oxyacetic acid, including copolymers derived from poly(alkylene 1,4-phenylene-bis-oxyacetate) and glycolide and/or lactide.

U.S. Patent No. 2,516,955 (Butler et al.) discloses esters of phenylene-bis-oxyacetic acid and monohydric alcohols.

Low molecular weight polyesters of phenylene-bis-oxyacetic acid are claimed to have been produced by Spanagel and Carouthers, as reported in JACS, 57, pages 935-936 (1935).

Doddi et al., in U.S. Patent No. 4,052,988, disclose synthetic absorbable sutures and other surgical devices produced from polymers of p-dioxanone.

Detailed Description of the Invention

The two types of polymer that are used in making the blends of the invention are known. Preferred methods for producing these polymers are illustrated below in Examples 1-3.

Example 1

Preparation of Polyester of Ethylene Glycol and 1,4-Phenylene-bis-oxyacetic acid

To a flame dried, mechanically stirred, 1 liter glass reactor, suitable for polycondensation reactions, is charged 127.1 grams of dimethyl 1,4-phenylene-bis-oxyacetate (0.5 moles), 62.1 grams ethylene glycol (1.0 mole) and 9.0 milligrams dibutyltin oxide (0.0071 weight

percent based on the expected polymer weight). After purging the reactor and venting with nitrogen the reactor is immersed in a silicone oil bath and connected to a gas supply to maintain nitrogen at 1 atmosphere of pressure. The stirred mixture is heated to and maintained at 160°C., 190°C., and 210°C. for 2, 1, and 2 hours respectively during which time the methanol produced in the reaction, along with some ethylene glycol, is distilled from the reaction mixture and collected. The reactor is allowed to cool to room temperature. Some time later the reactor is evacuated and heated; temperatures of 190°C., 210°C., and 220°C. are maintained for 1, 1, and 2 hours respectively. The collection of distillate is continued during the low pressure (less than about 100 microns) stage of the polymerization. The temperature is increased from 220°C. to 240°C. over the course of 30 minutes and 240°C. is maintained for 3 hours. The reactor is removed from the oil bath and allowed to cool. The formed polymer is isolated, ground and dried. The polymer has an inherent viscosity of 1.31 dl/g as determined in hexafluoroisopropyl alcohol ("HFIP") at 25°C. and a concentration of 0.1 g/dl.

Lower molecular weight polymers can be easily produced by decreasing the reaction time at 240°C. or by decreasing the final polymerization temperature. These techniques are well known to those skilled in the art of polycondensation reactions.

The polymers may also be produced in a three stage polymerization where the diacid moiety, the diol and a catalyst are heated at atmospheric temperatures (under nitrogen) in a molten state, followed by reaction at reduced pressure in a molten state to produce relatively low molecular weight species of the polymer. The low molecular weight material is pelletized or ground and

crystallized. The material is heated under vacuum in a third stage at a temperature below its melting point. This last solid state polymerization stage increases the molecular weight significantly.

Example 2

Preparation of Polyester of Ethylene Glycol and 1,4-Phenylene-bis-oxyacetic acid

To a flame dried, mechanically stirred, 250 ml reactor, suitable for polycondensation, is charged 60.0 grams of dimethyl 1,4-phenylene-bis-oxyacetate (0.236 mole), 31.6 grams ethylene glycol (0.509 mole) and 11.7 milligrams of dibutyltin oxide (0.0197 weight percent based on expected polymer weight). After purging the reactor and venting with nitrogen, the reactor is immersed in a silicone oil bath and further connected to a gas supply to maintain nitrogen at one atmosphere of pressure. The stirred mixture is heated to and maintained at 190°C. for 7 hours during which time the formed methanol, along with some ethylene glycol, is distilled from the reaction mixture and collected. The pressure in the reactor is reduced and a temperature of 190°C. maintained for an additional 3 hours under high vacuum. The stirred reaction mass is maintained under vacuum at 200°C. and 220°C. for 2 and 7 hours respectively while continuing to remove distillates. The polymer is subjected to 100°C. for 3 hours during which time the polymer crystallizes. The polymer is isolated, ground (particle size less than 3 mm) and dried. The polymer has an inherent viscosity of 0.76 dl/g measured in HFIP at 25°C. and a concentration of 0.1 g/dl.

Example 3

Preparation of Poly(p-dioxanone) Homopolymer (Typical Laboratory-Scale Preparation)

Under a dry nitrogen atmosphere, p-dioxanone (155.2 grams, 1.52 moles), 1-dodecanol (0.473 gram, 2.54 millimoles, 4.73 milliters of a 0.537 M toluene solution), and a catalytic amount of stannous octoate (0.115 milliliter of a 0.33 M toluene solution, 0.038 millimoles) were added to a flame and vacuum dried 500 milliliter glass ampoule, equipped with a magnetic stirring bar. The contents of the ampoule were exposed to vacuum at room temperature for several hours with intermittant dry nitrogen purges. The glass ampoule was sealed under partial vacuum, placed in a silicone oil bath, and its contents were subjected to elevated temperatures (with rapid magnetic mixing as melt viscosity allowed) for one minute at about 120°C., three hours at 90°C., and 96 hours at 80°C. The resulting polyester was isolated by immersing the glass ampoule in liquid nitrogen and shattering the surrounding glass with a heavy object. The glass-free polymer was ground in a Wiley mill and stored under vacuum for 16 hours at room temperature. The ground polymer was heated at 80°C. under a reduced pressure of 0.05 millimeter of mercury for 16 hours to remove any unreacted p-dioxanone. The polyester had an inherent viscosity of 1.79 dl/gm measured at 25°C. and a concentration of 0.1 gram/dl in hexafluoroisopropyl alcohol.

The poly(alkylene phenylene-bis-oxyacetate) used in the invention can be represented by Formula I:

$$\text{I} \quad -\!\!\!\left( G-O-CO-CHR-O-Ph-O-CHR-CO-O \right)\!\!\!-_n$$

wherein G represents the residue after removal of the hydroxyl groups of a dihydric alcohol, wherein Ph represents phenylene or alkyl- or alkoxy-substituted phenylene, preferably 1,4-phenylene, wherein each R individually represents hydrogen or lower alkyl such as methyl, ethyl, or propyl, and wherein n represents a number whose average value reflects the degree of polymerization of the polyester. Is is produced by reacting a dihydric alcohol with a phenylene bis-oxyacetic acid or, preferably, a lower alkyl diester thereof.

The dihydric alcohols, which can be used singly or in mixtures, that can be employed to produce the polyesters include $C_2$ to $C_8$ alkylene glycols such as ethylene glycol, 1,2- and 1,3-propylene glycol, 1,4-butylene glycol, 1,6-hexylene glycol, and the like; polyalkylene glycols such as diethylene glycol, triethylene glycol, poly(oxytetramethylene) glycol, and the like; cycloaliphatic diols such as 1,4-cyclohexanedimethanol, and the like; and aromatic dihydric alcohols such as 1,4-bis(2-hydroxyethoxy)benzene, and the like. The polymethylene glycols having two to six carbon atoms are preferred. Ethylene glycol is most preferred.

The dihydric alcohol and the diester (or half ester or diacid) are usually reacted in proportions of from about 1.1 to about 4 moles of dihydric alcohol per mole of diester (or half ester or diacid).

A catalytically effective amount of a transesterification catalyst, with or without an esterification catalyst, is used in the reaction. While the reaction would proceed with a wide variety of such catalysts, as a practical matter because the blends of the invention are intended

for use in absorbable products, biologically acceptable catalysts used in very small amounts are preferred. Specific examples of such catalysts are stannous octoate and dibutyltin oxide. Illustrative proportions are from about 750 to about 30,000, and preferably about 1500 to about 15,000 moles of phenylene-bis-oxyacetic acid or ester thereof per mole of catalyst.

The polyesters usually have molecular weights in excess of about 2000, and inherent viscosities of at least about 0.2 dl/gm, tested at a concentration of 0.1 gm/dl in hexafluoroisopropyl alcohol at 25°C.

The polydioxanone used in the invention will normally have an inherent viscosity of greater than about 0.5, and preferably greater than about 1.5 dl/gm, tested at a concentration of 0.1 gm/dl in hexafluoroisophropyl alcohol at 25°C. The preferred polydioxanone for use in the invention is poly(p-dioxanone) homopolymer.

The blends of the invention are produced by mixing the two polymers in a suitable mixer such as a single or twinscrew extruder, or a Helicon (helical twin blade) mixer, at elevated temperature of, e.g., about 140° to about 160°C. (i.e., a temperature above the $T_m$ of both polymeric components). The mixing is carried out for a relatively short period of time, sufficient to make a homogeneous blend (e.g., 5 to 20 minutes) of the two polymers that is a single phase composition, as determined by optical microscopic examination of the molten blend at about 140°C. The blend usually comprises from about 5 to 30, and preferably from about 5 to 20, weight per cent poly(alkylene phenylene-bis-oxyacetate), the remainder comprising polydioxanone. The blended polymers are then collected by conventional procedures, such as by pelletizing after the blending operation. One procedure

for carrying out the blending is illustrated in the following Examples 4-7:

Examples 4-7 and Control Example 1

Dyed poly(p-dioxanone) homopolymer ("PDO") having an inherent viscosity in HFIP of about 1.8, and poly(ethylene 1,4-phenylene-bis-oxyacetate) ("PEPBO") having an inherent viscosity in HFIP of 0.62 were blended in a 3/4-inch single screw extruder, in the proportions (by weight) set forth in Table I, below. The die diameter was 0.125 inch. The four temperature zones $T_1$ - $T_4$ listed in Table I refer to four successive temperature zones along the length of the extruder barrel. The extruder was a Brabender Prep Center Universal Mixer/Extruder equipped with a Model 2523 extruder barrel.

The speed of the melt being extruded from the die was 24-26 feet/minute ("FPM"). The extruded rod was quenched in water at the temperature indicated in the Table, and then taken up on a heated godet (155°F.), from which the rod was fed into a pelletizer. Table I, below, displays the conditions used in the blending operation:

TABLE I

MELT BLENDING OF PDO/PEPBO BLENDS

| Composition, PDO/PEPBO | Example | Temperatures(2) $T_1$ °C. | $T_2$ °C. | $T_3$ °C. | $T_4$ °C. | $T_{melt}$ °C. | Press. Screw, PSI | Quench Media/ Temp. °C. | Takeup, FPM(1) |
|---|---|---|---|---|---|---|---|---|---|
| 100/0 | Control 1 | 145 | 155 | 160 | 155 | 155 | 1000 | water/21 | 24 |
| 95/5 | Example 4 | " | " | " | " | " | 1000 | water/22 | 24 |
| 90/10 | Example 5 | " | " | " | " | " | 1100 | water/22 | 24 |
| 85/15 | Example 6 | " | " | " | " | " | 1300 | water/22 | 25 |
| 80/20 | Example 7 | " | " | " | " | " | 1350 | water/23 | 26 |

(1) FPM - Feet per Minute
(2) $T_1$ - Feed zone
$T_2$ - Transition zone
$T_3$ - Compression zone
$T_4$ - Die
$T_{melt}$ - Melt temperature in the die.

EXTRUSION

The blends are melt extruded through a spinnerette in a conventional manner to form one or more filaments.

Extrusion of the blends (Examples 4-7, and Control 1) was accomplished using an INSTRON Capillary Rheometer. The blends were packed in a 90°C. preheated extrusion chamber and extruded through a 40 mil die (L/D=24.1) after a dwell time of 10 to 11 minutes at the extrusion temperature using a ram speed of 2 cm/min. While extrusion temperatures depend both on the polymer Tm and on the melt viscosity of the material at a given temperature, extrusion of the blends at temperatures of about 40° to 75°C. above the Tm is usually satisfactory. The extrusion temperatures of the blends described herein ranged from 155° to 175°C. The extrudate was taken up through an ice water quench bath at 24 feet/minute. (A screw-type extruder or similar device can be substituted for the INSTRON Capillary Rheometer.)

The extrudate filaments are subsequently drawn about 5.75X to 6X in a multistage drawing process in order to achieve molecular orientation and improve tensile properties. The extrudates described herein were drawn 2 hours to about 1 week after extrusion. (The length of time elapsed between extrusion and drawing may affect the drawing process; the optimum time elapsed is easily determined by simple experimentation for each fiber composition.) The manner of drawing is as follows:

The extrudate (diameter range, 17.5-18.5 mils) passed through rollers at an input speed of four feet per minute and into a heated draw bath of glycerine. The temperatures of the draw bath can vary from about 25° to 90°C.; the examples described herein employ a temperature of

52°C. The draw ratio in this first stage of drawing can vary from 3X to about 7X; the examples described herein employ draw ratios from 4X to 5X. The partially drawn fibers are then placed over a second set of rollers into a glycerine bath (second stage) kept at temperatures ranging from 50° to 95°C.; the examples described herein employ a second stage draw temperature of 75°C. Draw ratios of up to 2X are applied in this second stage, but a ratio range of from 1.2X to 1.5X has been employed in the examples. The fiber is passed through a water-wash, taken up on a spool, and dried. A set of hot rollers can be substituted for a portion or all of the glycerine draw bath. The resulting oriented filaments have good straight and knot tensile strengths.

Dimensional stability and tensile strength retention of the oriented filaments may be enhanced by subjecting the filaments to an annealing treatment. This optional treatment consists of heating the drawn filaments to a temperature of from about 40° to 90°C., most preferably from about 60° to 80°C. while restraining the filaments to prevent any substantial shrinkage. This process may begin with the filaments initially under tension or with up to 20% shrinkage allowed prior to restraint. The filaments are held at the annealing temperature for a few seconds to several days or longer depending on the temperature and processing conditions. In general, annealing at 60° to 80°C. for up to about 24 hours is satisfactory for the blends of the invention. Annealing is preferably carried out under nitrogen or other inert atmosphere. Optimum annealing time and temperature for maximum fiber *in vivo* strength retention and dimensional stability is readily determined by simple experimentation for each fiber composition.

The characteristic properties of the filaments of the invention are readily determined by conventional test procedures. The tensile properties (i.e., straight and knot tensile strengths, Young's Modulus, and elongation) displayed herein were determined with an INSTRON tensile tester. The settings used to determine the straight tensile, knot tensile, break elongation, and Young's Modulus were the following, unless indicated:

| | Gauge Length (cm) | Chart Speed (cm/min) | Crosshead Speed (cm/min) |
|---|---|---|---|
| Straight Tensile | 12 | 20 | 10 |
| Knot Tensile | 5 | 10 | 10 |
| Break Elongation | 12 | 20 | 10 |
| Young's Modulus | 12 | 20 | 10 |

The straight tensile strength is calculated by dividing the force to break by the initial cross-sectional area of the fiber. The elongation to break is read directly from the stress-strain curve of the sample allotting 4-1/6% per centimeter of horizontal displacement.

Young's Modulus is calculated from the slope of the stress-strain curve of the sample in the linear elastic region as follows:

$$\text{Young's Modulus} = \frac{\tan\Theta \times GL \times CS \times SL}{XH \times XS}$$

Θ is the angle between the slope and the horizontal, XS is the initial cross-sectional area of the fiber, SL is the scale load, XH is the crosshead speed, CS is the chart speed, and GL is the gage length. The SL may be selected to provide a Θ close to 45°.

The knot tensile strength of a fiber is determined in separate experiments. The test article is tied into a surgeon's knot with one turn of the filament around flexible tubing of 1/4 inch inside diameter and 1/16 inch wall thickness. The surgeon's knot is a square knot in which the free end is first passed twice, instead of once, through the loop, and the ends drawn taut so that a single knot is superimposed upon a compound knot. The first knot is started with the left end over the right end and sufficient tension is exerted to tie the knot securely.

The specimen is placed in the INSTRON tensile tester with the knot approximately midway between the clamps. The knot tensile strength is calculated by dividing the force required to break by the initial cross-sectional area of the fiber.

The blends identified above as Examples 4-7, and Control Example 1 (i.e., 100% PDO) were spun into filaments. The two Tables below, II and III, display the inherent viscosities of the blends, their melting points, certain extrusion and drawing conditions, and selected properties of the drawn and oriented filaments.

TABLE II

PDO/PEPBO MONOFILAMENT PROCESSING

| Example | Composition PDO/PEPBO | IV in HFIP | Tm, °C. | Ext. Temp., °C. | Melt Viscosity, poise | Shear Rate, $sec^{-1}$ |
|---|---|---|---|---|---|---|
| Control 1 | 100/0 | 1.87 | 104-107 | 150 | 9030 | 213 |
| Example 4 | 95/5 | 2.18 | 105-109 | 175 | 7410 | 213 |
| Example 5 | 90/10 | 2.04 | 104-109 | 170 | 7310 | 213 |
| Example 6 | 85/15 | 2.05 | 104-108 | 160 | 7740 | 213 |
| Example 7 | 80/20 | 1.76 | 105-120 | 155 | 7410 | 213 |

TABLE III

PDO/PEPBO MONOFILAMENT PROPERTIES*

| Example | Draw Ratio @ Temp., °C. | Dia., mils | Straight Tensile, psi | Elong., % | Young's Modulus, psi | Knot Tensile, psi |
|---|---|---|---|---|---|---|
| Control 1 | 6x (4x/52+1.5x/75) | 7.7 | 86,100 | 52 | 255,000 | 55,600 |
| Example 4 | 6x (4x/52+1.5x/75) | 7.4 | 92,100 | 51 | 232,500 | 53,700 |
| Example 5 | 5.75x (4x/52+1.44x/75) | 7.5 | 89,400 | 55 | 161,000 | 47,500 |
| Example 6 | 6x (5x/52+1.2x/75) | 7.6 | 82,900 | 57 | 198,000 | 51,400 |
| Example 7 | 6x (5x/52+1.2x/75) | 7.6 | 72,100 | 63 | 151,500 | 48,300 |

*Unannealed

Examples 8-9

An Atlantic 2 CV reactor (a Helicon mixer) was first thoroughly dried by heating to 100°C. for 16 hours under a reduced pressure of 0.05 millimeter of mercury. Once cooled to room temperature, the reactor was vented with nitrogen and 10 grams of PEPBO having an inherent viscosity of 0.2, as measured in HFIP at 25°C. and a concentration of 0.1 gm/dl, in addition to 90 grams of undyed, poly(p-dioxanone) having an inherent viscosity in HFIP of about 1.8, were quickly added.

The charged reactor was then subjected to 0.05 millimeter of mercury pressure at room temperature for 1.0 hour, during which time it was thrice purged with nitrogen and subsequently evacuated to reduced pressure. Under a positive pressure of nitrogen, the reactor was next heated to and maintained for the duration of the experiment at 150°C. After 0.25 hour at 150°C., the charge had melted. Mechanical mixing at 8 RPM was then begun and continued for the duration of the blending experiment. Approximately 40 grams of blend were discharged from the bottom of the reactor after the mixing times noted below.

Upon removal from the reactor, the blend samples were cooled to room temperature in a nitrogen atmosphere, chilled in liquid nitrogen, and then ground in a Wiley mill. The ground blend samples were dried at room temperature for 16 hours under a reduced pressure of 0.05 millimeter of mercury. The inherent viscosities of the dried blends, which are given below, were determined in HFIP at 25°C. and a concentration of 0.1 g/dl. The melting points of the dried blends were determined by thermal microscopy and are also given below.

| Example No. | Blending Time, Min. | I.V. | Tm, (°C.) |
|---|---|---|---|
| 8 | 10 | 1.39 | 101-106 |
| 9 | 20 | 1.39 | 102-106 |

Examples 10-11

An Atlantic 2 CV reactor was first thoroughly dried by heating to 100°C. for 16 hours under a reduced pressure of 0.05 millimeter of mercury. Once cooled to room temperature, the reactor was vented with nitrogen and 10 grams of PEPBO having an inherent viscosity of 0.49, measured in HFIP at 25°C. and a concentration of 0.1 g/dl, in addition to 90 grams of undyed poly(p-dioxanone) having an inherent viscosity in HFIP of about 1.8, were quickly added. The charged reactor was then subjected to 0.05 millimeter of mercury pressure at room temperature for 1.0 hour, during which time it was thrice purged with nitrogen and subsequently evacuated to reduced pressure. Under a positive pressure of nitrogen, the reactor was next heated to and maintained for the duration of the experiment at 150°C. After 0.25 hour at 150°C. the charge had melted. Mechanical mixing at 8 RPM was then begun and continued for the duration of the blending experiment. Approximately 40 grams of blend were extruded from the reactor after the mixing times noted below.

Upon removal from the reactor, the blend samples were cooled to room temperature in a nitrogen atmosphere, chilled in liquid nitrogen, and then ground in a Wiley mill. The ground blend samples were dried at room temperature for 16 hours under a reduced pressure of 0.05 millimeter of mercury. The inherent viscosities of the dried blends, which are given below, were determined in HFIP at 25°C. and a concentration of 0.1 g/dl. The

melting points of the dried blends were determined by thermal microscopy and are also given below.

| Example No. | Blending Time, min. | I.V. | Tm., (°C.) |
|---|---|---|---|
| 10 | 10 | 1.49 | 102-105 |
| 11 | 20 | 1.46 | 103-106 |

Controls 2 and 3

An Atlantic 2 CV reactor was thoroughly dried by heating to 100°C. for 16 hours under a reduced pressure of 0.05 millimeter of mercury. Once cooled to room temperature, the reactor was vented with nitrogen and 100 grams of undyed poly(p-dioxanone) having an inherent viscosity in HFIP of about 1.8, were quickly added.

The charged reactor was then subjected to 0.05 millimeter of mercury pressure at room temperature for 1.0 hour, during which time it was thrice purged with nitrogen and subsequently evacuated back to reduced pressure. Under a positive pressure of nitrogen, the reactor was next heated to and maintained for the duration of the experiment at 150°C. After 0.25 hour at 150°C. the charge had melted. Mechanical mixing at 8 RPM was then begun and continued for the duration of the blending experiment. Approximately 40 grams of sample were extruded from the reactor after the mixing times noted below.

Upon removal from the reactor, the control samples were cooled to room temperature in a nitrogen atmosphere, chilled in liquid nitrogen, and then ground in a Wiley mill. The ground control samples were dried at room temperature for 16 hours under a reduced pressure of 0.05 millimeter of mercury. The inherent viscosities of

the dried controls, which are given below, were determined in HFIP at 25°C. and a concentration of 0.1 g/dl.

| Control No. | Mixing Time, (Min.) | I.V. |
|---|---|---|
| 2 | 10 | 1.65 |
| 3 | 20 | 1.62 |

Examples 12-23

Blend examples 4-7 were individually reblended in the Atlantic 2 CV reactor following the procedure given below.

An Atlantic 2 CV reactor was first thoroughly dried by heating to 100°C. for 16 hours under a reduced pressure of 0.05 millimeter of mercury. Once cooled to room temperature, the reactor was vented with nitrogen and one hundred grams of the blends of Examples 4-7 were quickly added. The charged reactor was then subjected to 0.05 millimeter of mercury pressure at room temperature for 0.5 hour, during which time it was thrice purged with nitrogen and subsequently evacuated back to reduced pressure. Under a positive pressure of nitrogen, the reactor was next heated to and maintained for the duration of the experiment at 150°C.

After 0.25 hour at 150°C. the charge had melted. Mechanical mixing at 15 RPM was then begun and continued for the duration of the blending experiment. Approximately 30 grams of blend were extruded from the reactor after the mixing times noted below:

| Original Example No. | Weight Composition (PDO/PEPBO) | Reblend Example No. | Reblending Time, Min. |
|---|---|---|---|
| 4 | 95/5 | 12 | 10 |
| | | 13 | 20 |
| | | 14 | 40 |
| 5 | 90/10 | 15 | 5 |
| | | 16 | 10 |
| | | 17 | 20 |
| 6 | 85/15 | 18 | 5 |
| | | 19 | 10 |
| | | 20 | 20 |
| 7 | 80/20 | 21 | 5 |
| | | 22 | 10 |
| | | 23 | 15 |

Upon removal from the reactor, blend samples were cooled to room temperature in a nitrogen atmosphere, chilled in liquid nitrogen, and then ground in a Wiley mill. The ground blend samples were first dried at room temperature for several hours under a reduced pressure of 0.05 millimeter of mercury, and then at 80°C. for 16 hours while still exposed to vacuum. The inherent viscosities of all dried reblend samples, which are given below, were determined in HFIP at 25°C. and a concentration of 0.1 g/dl. The melting points of most of the dried reblend samples were determined by thermal microscopy and are also given below.

| Reblend No. | I.V. | Tm., °C. |
|---|---|---|
| 12 | 1.80 | 104-110 |
| 13 | 1.74 | 104-110 |
| 14 | 1.75 | 103-110 |
| 15 | 1.84 | - |
| 16 | 1.87 | - |
| 17 | 1.83 | - |
| 18 | 1.64 | 105-110 |
| 19 | 1.64 | 104-110 |
| 20 | 1.61 | 104-110 |
| 21 | 1.58 | 104-110 |
| 22 | 1.56 | 104-110 |
| 23 | 1.55 | 105-110 |

Monofilaments made from the above-described blends of PDO and PEPBO (Examples 8-23) were extruded, drawn, and annealed under conditions analogous to those described above in detail with respect to Examples 4-7 and Control 1. Certain of the specific conditions are displayed below in Tables IV and V. Tables IV and V also display certain properties of the unannealed fibers (diameter, knot strength, straight tensile strength, elongation at break, and Young's modulus). The annealed filaments were cut to appropriate lengths, placed in individual paper folders and heat-sealable vented foil envelopes. The foil envelopes were subjected to 50°C. and 0.1 mm Hg pressure for 72 hours and then sealed under nitrogen. Portions of the packaged filaments were sterilized by exposure to 2.5 Mrads of gamma radiation from a $Co^{60}$ source. Table VI, below, displays representative properties of the annealed and sterilized filaments. (Control 4 is dyed PDO, IV = 1.8, that had not been subjected to additional reblending, as Controls 2 and 3 were.)

Part 1

TABLE IV

EXTRUSION, ORIENTATION, AND ANNEALING CONDITIONS, AND UNANNEALED FIBER PROPERTIES OF POLY(P-DIOXANONE)/PEPBO BLENDS

| Blend Example No. | Extrusion | | Orientation | | Annealing(2) | |
|---|---|---|---|---|---|---|
| | Temp, (°C.) | $\eta_{APP}$(1), (Poise) | 1st Stage (X/°C.) | 2nd Stage (X/°C.) | Time, hrs. | Temp, °C. |
| 12 | 160 | 8600 | 4/54 | 1.375/74 | 6.5 | 74 |
| 14 | 160 | 6980 | 4/54 | 1.375/73 | 6.25 | 74 |
| 15 | 160 | 8490 | 4/53 | 1.5/72 | 6.25 | 74 |
| 16 | 170 | 6880 | 4/54 | 1.5/72 | 6.25 | 72 |
| 19 | 160 | 7520 | 4/54 | 1.375/74 | 6.25 | 74 |
| 20 | 160 | 5210 | 4/55 | 1.375/73 | 6.5 | 74 |
| 21 | 160 | 4890 | 4/55 | 1.375/74 | 6.5 | 74 |
| 22 | 160 | 5640 | 4/54 | 1.375/73 | 6.5 | 74 |
| 23 | 160 | 6020 | 4/55 | 1.375/74 | 6.5 | 74 |
| Control 4 (pure PDO) | 150 | 11300 | 4/55 | 1.375/75 | 6.0 | 77 |

(1) Melt viscosity at a shear rate of 213 $sec^{-1}$.
(2) 5% Relaxation.

Part 2

TABLE IV

EXTRUSION, ORIENTATION, AND ANNEALING CONDITIONS, AND UNANNEALED FIBER PROPERTIES OF POLY(P-DIOXANONE)/PEPBO BLENDS

UNANNEALED FIBER PROPERTIES

| Blend Example No. | DIAM., (MIL) | Knot (PSI) | Straight (PSI) | Elong. (%) | Y.M. (PSI) |
|---|---|---|---|---|---|
| 12 | 8.2 | 38600 | 68550 | 83 | 160000 |
| 14 | 8.2 | 39600 | 64600 | 78 | 169000 |
| 15 | 7.7 | 49200 | 67200 | 65 | 158000 |
| 16 | 7.7 | 50700 | 76700 | 73 | 174000 |
| 19 | 8.1 | 37300 | 60900 | 95 | 118000 |
| 20 | 8.5 | 32100 | 54800 | 101 | 100000 |
| 21 | 8.3 | 35500 | 47500 | 93 | 93700 |
| 22 | 8.1 | 34500 | 51800 | 101 | 101000 |
| 23 | 8.1 | 35900 | 51800 | 95 | 122000 |
| Control 4 | 8.1 | 47200 | 69300 | 56 | 213000 |

Part 1

TABLE V

EXTRUSION, ORIENTATION, AND ANNEALING CONDITIONS, AND UNANNEALED FIBER PROPERTIES OF POLY(P-DIOXANONE)/PEPBO BLENDS

| Blend Example No. | Extrusion | | Orientation | | Annealing[2] | |
|---|---|---|---|---|---|---|
| | Temp, (°C.) | $\eta_{App}$[1], (Poise) | 1st Stage (X/°C.) | 2nd Stage (X/°C.) | Time, hrs. | Temp, °C. |
| 10 | 150 | 6177 | 4/50 | 1.5/70 | 6.0 | 80 |
| 11 | 150 | 5748 | 4/52 | 1.5/70 | 6.0 | 80 |
| 8 | 150 | 4136 | 4/50 | 1.5/72 | 6.0 | 80 |
| 9 | 150 | 3975 | 4/50 | 1.5/72 | 6.0 | 80 |
| Control 2 | 150 | 7252 | 4/51 | 1.5/71 | 6.0 | 80 |
| Control 3 | 150 | 8057 | 4/50 | 1.5/70 | 6.0 | 80 |

(1) At a shear rate of 213 $sec^{-1}$.
(2) 5% Relaxation

Part 2

TABLE V

EXTRUSION, ORIENTATION, AND ANNEALING CONDITIONS, AND UNANNEALED FIBER PROPERTIES OF POLY(P-DIOXANONE)/PEPBO BLENDS

Unannealed Fiber Properties

| Blend Example No. | Diam., (Mil) | Knot (PSI) | Straight (PSI) | Elong (%) | Y.M. (PSI) |
|---|---|---|---|---|---|
| 10 | 7.9 | 43450 | 68300 | 60 | 186000 |
| 11 | 7.9 | 43450 | 73400 | 64 | 192000 |
| 8 | 7.7 | 42700 | 60600 | 60 | 132000 |
| 9 | 7.9 | 45700 | 68750 | 73 | 144000 |
| Control 2 | 8.1 | 43700 | 73350 | 48 | 252000 |
| Control 3 | 8.5 | 50200 | 73100 | 55 | 273000 |

ETH

TABLE VI

FIBER PROPERTIES OF ANNEALED $Co^{60}$ STERILIZED PDO/PEPBO MELT BLEND MONOFILAMENTS

| Example No. | Weight Composition PDO/PEPBO | Blend Time (min.) | Fiber I.V. | % Cryst.** (X-ray) | Dia. (mil.) | Str. (psi) | Elong. (%) | Y.M. (psi) |
|---|---|---|---|---|---|---|---|---|
| 12* | 95/5 | 10 | 1.34 | 20 | 8.1 | 54,800 | 63 | 222,000 |
| 14* | | 40 | 1.31 | 20 | 8.4 | 52,600 | 60 | 199,000 |
| 15* | 90/10 | 5 | 1.37 | 22 | 7.8 | 58,400 | 43 | 239,000 |
| 16* | | 10 | 1.31 | 19 | 7.8 | 54,500 | 36 | 231,000 |
| 10 | | 10 | 1.32 | 22 | 7.9 | 55,900 | 50 | 263,000 |
| 11 | | 20 | 1.27 | 30 | 8.0 | 56,200 | 56 | 244,000 |
| 8 | | 10 | 1.18 | | 7.6 | 58,700 | 56 | 272,000 |
| 9 | | 20 | 1.10 | | 8.3 | 54,100 | 56 | 230,000 |
| 19* | 85/15 | 10 | 1.26 | 18 | 8.4 | 46,300 | 71 | 154,500 |
| 20* | | 20 | 1.22 | 18 | 8.0 | 45,400 | 69 | 177,000 |
| 21* | 80/20 | 5 | 1.17 | 19 | 8.2 | 41,700 | 75 | 175,000 |
| 22* | | 10 | 1.16 | 19 | 8.2 | 43,200 | 76 | 162,000 |
| 23* | | 15 | 1.20 | 20 | 8.3 | 40,900 | 72 | 171,000 |
| CONTROL 4* | 100/0 | 0 | 1.30 | 25 | 8.5 | 55,900 | 57 | 241,000 |
| 2 | | 10 | 1.34 | 24 | 8.3 | 61,900 | 49 | 269,000 |
| 3 | | 20 | 1.31 | 22 | 8.4 | 60,800 | 44 | 271,000 |

* - Dyed
** - After annealing, before irradiation

BREAKING STRENGTH RETENTION:

The breaking strength retention (BSR) of a fiber is determined by implanting two strands of the fiber in the dorsal subcutis of each of a number of Long-Evans rats. The number of rats used is a function of the number of implantation periods, employing 4 rats per period giving a total of eight (8) examples for each of the periods. Thus 16, 24, or 32 segments of each fiber are implanted corresponding to two, three, or four implantation periods. The periods of in vivo residence are 7, 14, 21, or 28 days. The ratio of the mean value of 8 determinations of the breaking strength (determined with an INSTRON Tensile tester employing the following settings: a gage length of 1 inch, a chart speed of 1 inch/minute, and a crosshead speed of 1 inch/minute) at each period to the mean value (of 8 determinations) obtained for the fiber prior to implantation constitutes its breaking strength retention for that period.

The in vivo BSR of certain of the annealed and cobalt-60 sterilized monofilaments identified above are displayed below in Table VII:

TABLE VII

IN VIVO BSR OF $Co^{60}$ STERILIZED PDO/PEPBO MELT BLEND MONOFILAMENTS

| Example No. | Weight Composition PDO/PEPBO | Blend Time (min.) | Sterilized Fiber I.V. | % Cryst.** (X-ray) | Dia. (mil.) | Initial Strength (lbs.) | % BSR 14 | 21 | 28 days |
|---|---|---|---|---|---|---|---|---|---|
| 4* | 100/0 | 0 | 1.30 | 25 | 8.5 | 3.28 | 70 | 67 | 54 |
| 14* | 95/5 | 40 | 1.31 | 20 | 8.4 | 2.95 | 77 | 68 | |
| 16* | 90/10 | 10 | 1.31 | 19 | 7.8 | 3.01 | 79 | 75 | |
| 20* | 85/15 | 20 | 1.22 | 18 | 8.0 | 2.50 | 73 | 69 | |
| 23* | 80/20 | 15 | 1.20 | 20 | 8.3 | 2.21 | 72 | 68 | |
| 10 | 90/10 | 10 | 1.32 | 22 | 7.9 | 2.44 | | 79 | 61 |
| 11 | 90/10 | 20 | 1.27 | 30 | 8.0 | 2.39 | | 85 | 51 |
| 8 | 90/10 | 10 | 1.18 | -- | 7.6 | 2.44 | | 81 | 60 |
| 9 | 90/10 | 20 | 1.10 | -- | 8.3 | 2.56 | | 76 | 62 |
| Control 2 | 100/0 | 10 | 1.34 | 24 | 8.3 | 3.06 | | 48 | 35 |
| Control 3 | 100/0 | 20 | 1.31 | 22 | 8.4 | 3.09 | | 55 | 32 |

* - Dyed
** - After annealing, before irradiation

## GENERATION OF ABSORPTION DATA

Under aseptic conditions, two 2-centimeter segments of a suture sample are implanted into the left and right gluteal muscles of female Long-Evans rats. Two rats per period are implanted for each of the examination periods. The animals utilized in these studies are handled and maintained in accordance with the requirements for the Animal Laboratory Welfare Act and its 1970 Amendment. The rats are killed at the appropriate periods by carbon dioxide asphyxiation, then their gluteal muscles are excised and fixed in buffered formalin. Utilizing standard histologic techniques, H and E stained slides of the muscles and implanted sutures are prepared for microscopic examination. Utilizing an ocular micrometer, the approximate suture cross-sectional area is estimated in each site. The cross-sectional area at five days is used as the reference value for estimating percent cross-sectional area remaining at subsequent intervals.

The absorption profiles of certain of the annealed and cobalt-60 sterilized monofilaments were determined. The results are displayed below in Table VIII:

TABLE VIII

IN VIVO ABSORPTION OF $CO^{60}$ STERILIZED PDO/PEPBO MELT BLEND MONOFILAMENTS

| Example No. | Weight Comp. PDO/PEPBO | Blend Time (Min @ 150°C.) | Sterilized Fiber I.V. | Dia. (mil.) | % Area Remaining | | | | |
|---|---|---|---|---|---|---|---|---|---|
| | | | | | 5 | 120 | 154 | 182 | 210 (days) |
| 10 | 90/10 | 10 | 1.32 | 7.9 | 100 | - | 6 | 0 | - |
| 8 | 90/10 | 10 | 1.18 | 7.6 | 100 | - | 74 | 0 | - |
| Control 2 | 100/0 | 10 | 1.34 | 8.3 | 100 | - | 0 | 0 | - |
| 11 | 90/10 | 20 | 1.27 | 8.0 | 100 | - | 15 | 0 | - |
| 9 | 90/10 | 20 | 1.10 | 8.3 | 100 | - | 29 | 0 | - |
| Control 3 | 100/0 | 20 | 1.31 | 8.4 | 100 | - | 4.5 | 0 | - |
| 12 | 95/5 | 10 | 1.34 | 8.1 | 100 | - | 0 | 0 | 0 |
| 16 | 90/10 | 5 | 1.37 | 7.8 | 100 | - | 14 | 1 | 0 |
| 19 | 85/15 | 10 | 1.26 | 8.4 | 100 | - | 34 | 12 | 0 |
| 22 | 80/20 | 10 | 1.16 | 8.2 | 100 | - | 56 | 30 | 11 |
| Control 4 | 100/0 | 0 | 1.30 | 8.5 | 100 | 96 | 7 | 0 | - |

**CLAIMS**

1. A blend of polydioxanone and poly(alkylene phenylenebis-oxyacetate), said blend being single phase by microscopic examination of the melt.

2. The blend of Claim 1, wherein said polydioxanone is present in the blend in proportions of from about 5 to about 30 per cent, by weight, based on weight of the blend.

3. The blend of Claim 1 or Claim 2 wherein said polydioxanone is poly(p-dioxanone) homopolymer.

4. The blend of any one of claims 1 to 3, wherein said phenylene is 1,4-phenylene.

5. The blend of any one of Claims 1 to 4, wherein said alkylene is ethylene.

6. A drawn and oriented surgical filament comprising the blend of any one of Claims 1 to 5.

7. The surgical filament of Claim 6, attached to a needle.

8. The surgical filament of Claim 6 or Claim 7, in sterile condition.

9. An absorbable, radiation sterilizable composition comprising the blend of any one of Claims 1 to 5.

10. A surgical device comprising the blend of any one of Claims 1 to 5.

11. The surgical device of Claim 10 in the form of a surgical staple.